# EUROPEAN PATENT SPECIFICATION

(11) **EP 1 476 081 B1**
(45) Date of publication and mention of the grant of the patent: **11.01.2012**
(21) Application number: 02806427.7
(22) Date of filing: 28.08.2002
(51) Int. Cl.: A61B 17/00

(54) **METHOD AND PROCESS FOR PRODUCING YOUTHFUL-APPEARING, SMALL-PORED, AND SMOOTH SKIN**
METHODE UND VERFAHREN ZUR ERZEUGUNG JUGENDLICH ERSCHEINENDER KLEINPORIGER UND GLATTER HAUT
PROCEDE ET PROCEDURE PERMETTANT DE LAISSER LA PEAU SOUPLE, AVEC DE PETITS PORES ET UN ASPECT JEUNE

(30) Priority: 14.01.2002 US 47335
(43) Date of publication of application: 17.11.2004
(73) Proprietor: Parsons, Diana J., Berkeley, CA 94805 (US)
(72) Inventor: Parsons, Diana, Berkeley, Ca 94805 (US)
(74) Representative: Bentham, Andrew
(86) International application number: PCT/US2002/027570
(87) International publication number: WO 2003/059144

(56) References cited:
- WO-A2-03/017824
- US-A- 5 423 803
- DATABASE MEDLINE [Online] KYE Y.C.: 'Resurfacing of pitted facial scars with a pulsed ER:YAG laser', XP002970713 Retrieved from NCBI Database accession no. 9357495 & DERMATOLOGIC SURGERY vol. 23, no. 10, October 1997, pages 880 - 883
- DATABASE MEDLINE [Online] HO ET AL.: 'Laser resurfacing in pigmented skin', XP002970714 Retrieved from NCBI Database accession no. 7496671 & DERMATOLOGIC SURGERY vol. 21, no. 12, December 1995, pages 1035 - 1037

## Description

### BACKGROUND OF INVENTION

### 1. Field of the Invention

The present invention relates generally to laser treatment of the skin and, more particularly, to a laser treatment inducing a chronic wound in the high dermis, leaving the epidermis intact, with topical pretreatment and post-treatment of the skin with retinoic acid.

### 2. Technical Background

Laser light treatment of the skin is used to rejuvenate the skin, remove pigments and hair, and treat infection. In the field of dermatology and plastic surgery the use of lasers is principally based upon two types of mechanisms: a thermal effect where the laser light energy is converted into heat energy, or a mechanical effect where the laser light energy is converted into shockwaves in the skin.

Most laser treatments use thermal-based laser therapy to vaporize the superficial wrinkles and top layers of the skin so that new collagen and skin can be naturally provided in a healing response. The procedure is performed under either local or general anesthetic, takes a few hours to complete, and recovery takes one to two weeks. During the first week patients may suffer a sensation of intense heat on the skin. Severe bums can occur and result in permanent scarring. Bacterial and yeast infection have been reported and can also lead to scarring. Additional potential complications include changes in pigmentation and herpes infection.

Retinoic acid (Retin-A, tretinoin) has also been used as a topical dermal treatment to improve the appearance and texture of the skin. Retinoic acid thins the stratum corneum, increases the thickness of the epidermis, and increases the production of collagen in the dermis. However, retinoic acid causes skin redness and sensitivity to the sun. Repeated use can cause a loss of pigment, painful irritation, dryness, swelling of the skin, and contact dermatitis.

In an effort to overcome these drawbacks of laser therapy and to avoid the unwanted side effects of retinoic acid, Tankovich et al. (U.S. Patent No. 6,036,684) developed a laser method of photomechanical activation in the skin using a Q⁻ switched Nd:YAG laser with a wavelength of 1064nm and an exposure of 2.5 J/cm². Exposure of the skin to this type of laser treatment by itself has no effect on the skin because the skin has no inherent target at the 1064nm, wavelength However, when an activating solution of graphite or carbon particles suspended in baby oil is applied to the skin, these particles become the target of this 1064nm wavelength, exploding when exposed to the laser light. Prior to application of the laser, the particles are forced into the skin, below the surface of the stratum corneum using ultrasound. Thereafter, the explosion of the carbon particles by the laser light produces a localized mechanical injury in the hair follicles and pores of the skin. There is no significant injury to the skin tissue because the laser energy which is not absorbed in the carbon is harmlessly dissipated in the skin. The low fluence of 2.5 J/cm² leaves the epidermis intact and the typical adverse effects of laser treatment do not occur.

Although the photomechanical laser process of *Tankovich* is considerably safer than the standard photothermal laser treatments and leaves the epidermis intact, this photomechanical laser process is relatively ineffective in treating the skin because the extent of injury in the high dermis resulting from the exploding carbon particles in the epidermis is insufficient to induce adequate collagen deposition during wound healing. What is needed, therefore, is means for enhancing the collagen deposition in the high dermis during the wound healing process.

### SUMMARY OF THE INVENTION

The present invention provides a process and method for producing skin rejuvenation and therapy by pretreating the skin with retinoic acid, producing a chronic thermal injury wound in the high dermis using photomechanical laser therapy, maintaining skin rejuvenation and therapy chronically by repeating the thermal injury in the high dermis at least once per year, and by topically applying retinoic acid bi-weekly. A Q-switched Nd:YAG laser with a wave length of 1064nm is used at 2.5 J/cm² to vaporize a topical activating solution of carbon particles suspended in baby oil. The energy of the laser laser is sufficient to cause the particles to explode. A single treatment of the face can be completed in 4 minutes without anesthesia, with no need for a period of recovery, and at a relatively low cost.

An advantage of the present invention is the production of a chronic wound in the high dermis with no damage to the epidermis.

Another advantage of the present, invention is the enhancement of laser-induced collagen deposition in the high dermis with retinoic acid.

Another advantage of the present invention is the chronic life-time rejuvenation and therapy of the skin.

Another advantage of the present invention is the removal and prevention of acne by suppression of sebaceous glands and reduction of skin pore size.

Another advantage of the present invention is laser therapy at a low energy level.

Another advantage of the present invention is the use of retinoic acid without side effects.

Another advantage of the present invention is therapeutic removal of infections of the skin.

Another advantage of the present invention is the chronic rejuvenation and therapy of the skin at a relatively low cost.

Another advantage of the present invention is that a single treatment of the skin of the face can be completed within four minutes.

### BRIEF DESCRIPTION OF THE DRAWINGS

**Fig. 1** describes the method of the present invention
**Fig. 2** illustrates a cross sectional view of the skin and the role of heat and oil in the method of the present invention.
**Fig. 3** describes the process of the present invention.
**Fig. 4** shows the effects of the laser method of the present invention on the restoration of the skin of a patient suffering from acne vulgaris.

### DETAILED DESCRIPTION OF THE PREFERRED EMBODIMENTS

While the following description details the preferred embodiments of the present invention, it is to be understood that the invention is not limited in its application to the details of construction and arrangement of the parts illustrated in the accompanying drawings, since the invention is capable of other embodiments and of being practiced in various ways.

Wound healing in the skin occurs over a year's time after an initial wounding, regardless of the means by which the wounding is induced. In the first five days the wound is comprised of inflammatory cells and new blood vessels, Then an immature form of collagen is laid down parallel to the skin surface. For six months the body shifts this collagen around, trying to identify the strongest repair. Around six months after the initial wounding, this immature collagen is replaced with mature collagen protein oriented perpendicular to the skin surface. Three months into this process, or months ten, eleven, and twelve from the initial wounding, these protein strands cross-link. This effect is visible on the skin's surface as an apparent shrinkage of the size of the scar to one third its original size.

Twelve months from the initial injury, the wound becomes quiescent. However, any additional insult to the wound site during the first six months of repair causes a signal for a stronger permanent repair. Any additional insult to the wound site during the second six months results in additional mature collagen in the wound. As long as some injury is repeated before twelve months has elapsed, the site becomes a chronic wound producing more collagen, undergoing more cross-linking, and more total surface area shrinkage.

The method and process of the present invention described herein outlines a series of treatments using the *Tankovich* laser and activating solution to maximize the initial wounding signal during the first six months of application. The *Tankovich* processes are disclosed in U.S. Patent No. 5,423,803 and U.S. Patent No. 6,036,684 . However, in the present invention the activating solution does not need to be forced into the spaces of the skin with ultrasound as required by the *Tankovich* process. Beginning at the sixth month from the initial wounding, a series of booster laser treatments is given until the desired skin resurfacing end point is reached. At a minimum, one booster laser treatment is given before twelve months has elapsed to keep the wound active. If the wound is not kept active, it will be necessary to repeat the initial wounding series of the first six months in order to continue the resurfacing process chronically.

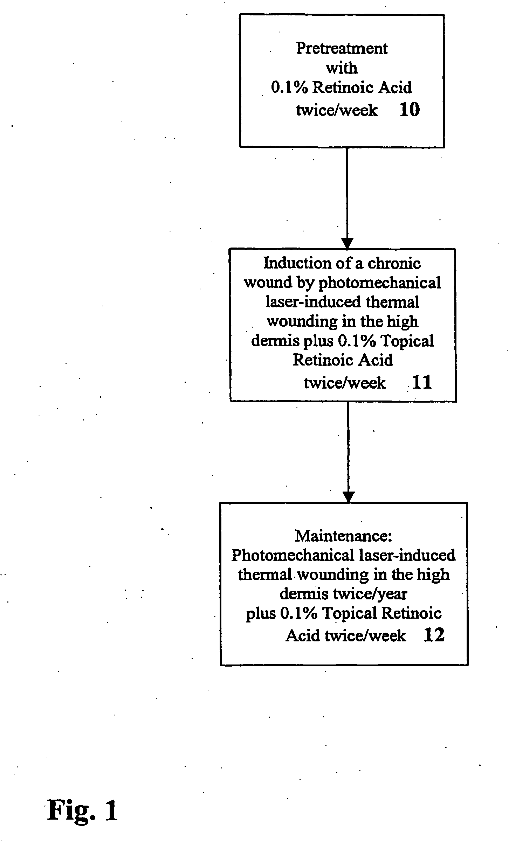

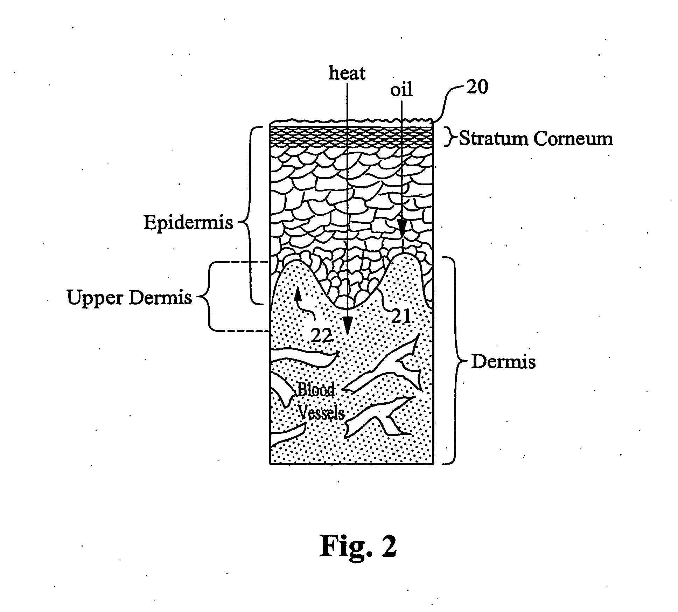

The first step of the method of the present invention is shown in **Fig. 1****, Step 10**, which is a topical pretreatment of the skin with a collagen inducing agent and an angiogenesis inducing agent, preferably, retinoic acid, and in particular its nongeneric form, such as Retin-A (tretinoin). Retinoic acid returns the architecture of the skin back to its more youthful form. As skin ages, the hill and valley nature of the dermal-epidermal junction **21** flattens (see **Fig. 2**). Topical retinoic acid restores the up and down pattern at the junction **21**. In youth, the basal cells which originate at the dermal-epidermal junction **21** create the layers of the epidermis every three weeks. With aging, this slows to four to six weeks. Topical retinoic acid on the skin returns the process to every three weeks. The top most layer of epidermal cells, the stratum corneum, is comprised of dead cells held together by a cell glue. With age, this layer thickens making the skin less fresh-appearing and making the pores larger. Topical retinoic acid directly attacks the cell glue, thinning the stratum corneum and making the pores smaller by reducing the amount of debris in them. In my studies I have discovered that 0.05% to 1% concentration of retinoic acid in a topical formulation has the property of inducing new collagen and new blood vessel formation in the high dermis. The pre-treatment period is one to four weeks, preferably two weeks with retinoic acid being applied twice a week with at least two days in between each application. The laser therapy of the present invention will not be effective without the pre-treatment application of topical retinoic acid.

The second step **11** in the method of the present invention shown in **Fig. 1** is the creation of a chronic wound in the high dermis. This is produced by a photomechanical laser treatment wherein the laser light does not interact directly with the skin but instead interacts with a contaminant in the skin. The contaminant has the properties of absorbing the laser light and exploding. The preferred contaminant is carbon or graphite particles in oil **20** which is applied to the skin (see **Fig. 2**). Once the contaminant or activating solution is applied to the skin, the laser treatment can begin. The energy from the laser is adjusted to be just sufficient to cause the particles to explode. As the particles explode, they cause the removal of the stratum corneum and the mineral oil **20** penetrates into the epidermis producing hydration of the epidermis by retarding the evaporation of water (see **Fig. 2**). The heat from the explosion of the contaminant particles will induce a photothermal injury in the rete peg area of the high dermis **22** initiating a normal wound healing process. In order to produce a sufficient degree of injury to the wound, the laser treatment is produced several times over a six month period, preferably six times over a six month period. During this first six months the retinoic acid is applied topically twice per week as described above.

The build up of new collagen in the high dermis **22** in response to the laser treatment and retinoic acid treatment thickens the skin and increases its turgor resulting in smaller skin pores. Broken blood vessels, angiomata, and ice pick scars or suture marks are also minimized, being crowded out by the new collagen. Easy bruisability of the skin is lessened by the collagen build up. Lips become redder, fine lines are minimized, and dark circles around the eyes are minimized. As the new collagen cross-links, major wrinkle lines, such as nasal labial folds and perioral and perioccular lines (caused by the underlying attachment of muscles to the deep dermis), marionette lines, sunken-in corners of the mouth, and forehead, temporal, and glabellar lines, appear to melt away into the new skin. The new skin has a glowing quality resulting from the pulsating nature of the laser injury and the high content of mineral oil in the activating solution. Retinoic acid and laser treatments work together to minimize bound differential pigmentation caused by sun damage and hormones (mylasma). Redness from inflammation, as in acne vulgaris, acne rosacea, or maturing scars or striae is also reduced.

Because of the closeness of the sebaceous glands to the skin surface and because of the new collagen formation which reduces the size of the skin pores, the method of process of the present invention is useful in the treatment of sebaceous gland disorders such as acne. The secretion from the sebaceous glands is reduced and the smaller skin pore size prevents bacteria from entering the skin pores. In addition, heat generated from the explosion of the contaminate particles is sufficient to kill bacteria, fungi, and viruses on the skin's surface or in the high dermis. This also allows treatment of conditions such as, for example, plantar warts, herpes cold sores, athletes foot, psoriasis, and excema.

The third step **12** in the method of the present invention, depicted in **Fig. 1**, is the long term continued chronic maintenance of the high dermis in a wounded condition in order to produce a sustained rejuvenation of the skin and treatment of skin conditions. This is accomplished by application of the laser treatment of the present invention at least once a year and preferably twice, along with the continued application of topical retinoic acid to the skin twice per week as described above.

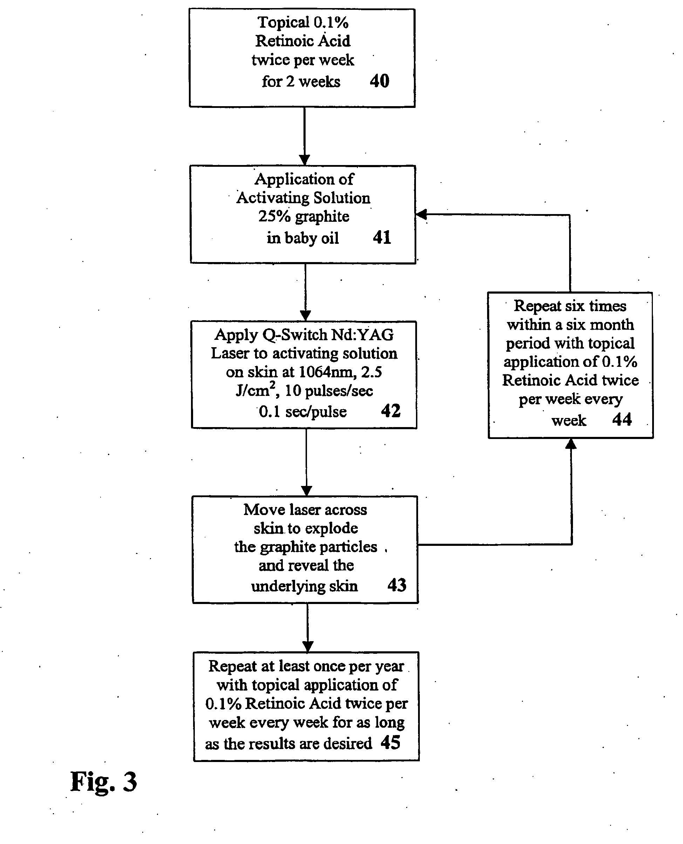

The process of the present invention for treating the skin is shown in **Fig. 3**. In the first step **40**, pre-treatment is initiated with topical retinoic acid applied to the skin at least twice per week. The concentration of retinoic acid in the topical formulation is between about 0.05% to 1%, preferably about 0.1%. The pretreatment period is, preferably, two weeks. In the next step **41**, an activating solution of graphite in baby oil is applied to the skin. The graphite-oil ratio may range from about 1:1 to 1:9, preferably 1:4, *i.e*., about 20 percent graphite suspended in about 80 percent oil by weight In the next steps **42** and **43** a laser beam is scanned over the area treated with the activating solution so as to clean substantially all of the mixture from the skin surface by exploding or fracturing the carbon or graphite particles in the oil. This scanning process takes from about 2 to 10 minutes to complete on the face, usually about 4 minutes.

It is preferred to use a Q-switched nepdymium:yttrium-aluminum-garnet (Nd:YAG) laser for the process of the present invention. The wave length of the radiation may range from about 800 nm to 2000 nm preferably about 1064 nm. The frequency of the pulses from the laser range from about 1 to 20 per second, preferably, about 10 per second. The duration of each pulse ranges from about 0.001 to 1 microsecond, preferably about 0.01 microsecond. The fluence or exposure of the skin treated with the activating solution ranges from about 1 to 3 J/cm², preferably, about 2.5 J/cm².

In the next step **44** the laser process is repeated several times to create a chronic wound in the high dermis **22** (see **Fig. 2**). The process may be repeated 2 to 12 times within a six month period, preferably six times. During this time retinoic acid is applied to the skin 1 to 4 times per week, preferably 2 times per week, with 2 to 3 days between applications. The application of retinoic acid may be varied in an equivalent manner on a monthly basis, *i.e*., 4 to 16 applications per month, preferably 8 applications per month, where the applications may all be applied within one or two weeks of a given month, but, preferably, applied each week of a month.

In the next step **45** the laser process is repeated at least once in the next six months and thereafter at least once per year, but, preferably twice per year. The yearly treatments will maintain a chronic wound in the high dermis. At the same time, topical retinoic acid is applied weekly to the skin as described above. The continued combination of intermittent laser therapy and topical retinoic acid will maintain the rejuvenation and therapeutic results in the skin indefinitely.

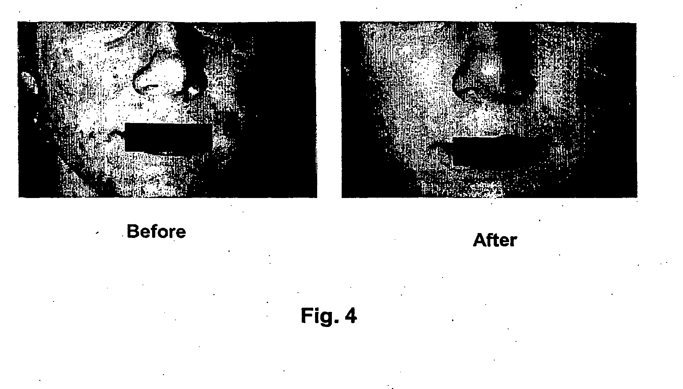

**Fig. 4** shows, by way of example, the effects of the laser method of the present invention on the skin of a patient suffering from acne vulgaris. The before picture presents the patient's skin prior to treatment and the after picture shows the patient's skin after the first six months of treatment of the skin with the method and process of the present invention. The lesions and scars are substantially reduced in severity and the process of acne formation has been completely prevented. Furthermore, this has been accomplished leaving the epidermis intact and normal. Continued chronic treatment with the method of the present invention is expected to provide further improvement in the appearance of the skin.

The foregoing description has been limited to specific embodiments of this invention. It will be apparent, however, that variations and modifications may be made by those skilled in the art to the disclosed embodiments of the invention, with the attainment of some or all of its advantages and without departing from the spirit and scope of the present invention. For example, agents other than retinoic acid which induce collagen and blood vessel growth may be used in place of retinoic acid. Any laser system capable of exploding or rupturing a contaminant in the skin without injuring the skin directly can be used in the present invention. The activating solution can be made with any suitable oil with any suitable contaminant.

## Claims

1. Retinoic acid for use in a method for treating the skin, said method comprising the steps of:
a) pretreating the skin with the retinoic acid;
b) producing a wound in the high dermis of the skin;
c) treating the skin chronically with said retinoic acid after producing said wound in the high dermis; and
d) maintaining the high dermis in a chronically wounded condition;
wherein the step of producing a wound in the high dermis is performed by exploding a contaminant on the surface of the skin with laser light.

2. Retinoic acid according to claim 1, wherein the method further comprises the steps of pretreating and chronically treating the skin with a blood vessel promoting agent.

3. Retinoic acid according to claim 1 or wherein said blood vessel promoting agent is retinoic acid.

4. Retinoic acid according to claim 1, wherein said retinoic acid is applied to the skin 1 to 4 times per week.

5. Retinoic acid according to claim 4, wherein said retinoic acid is applied to the skin 2 times per week.

6. Retinoic acid according to claims 4 or 5, wherein said retinoic acid is applied topically at a concentration of about 0.1%.

7. Retinoic acid according to any one of claims 3 to 6, wherein the pretreating step is performed for two weeks.

8. Retinoic acid according to my one of the preceding claims, wherein said production of a chronic wound in the high dermis leaves the epidermis intact.

9. Retinoic acid according to any one of the preceding claims, wherein the step of producing a chronic wound in the high dermis is performed by exploding a contaminant on the skin with laser light repeatedly within a six month period of said pretreating step and/or the step of maintaining a chronic wound in the high dermis in a chronically wounded condition is performed by exploding a contaminant on the surface of the skin with laser light at least once every twelve months.

10. Retinoic acid according to any one of the preceding claims, wherein producing a wound in the high dermis is completed in two to ten minutes or about four minutes.

11. Retinoic acid according to any one of the preceding claims, wherein the steps of producing and maintaining a chronic wound in the high dermis are performed by exploding a contaminant on the skin with a laser light having a wave length of from 800nm to 2000nm, a pulse frequency of from 1 to 20 per second, a pulse duration of from 0.001 to 1 microsecond, and a fluence of from 1 to 3 J/cm².

12. Retinoic acid according to claim 11, wherein said laser light has a wave length of about 1064nm, a pulse frequency of about 10 per second, a pulse duration of about 0.01 microsecond, and a fluence of about 2.5 J/cm², and optionally said laser light is produced by a Q-switched Nd:YAG laser.

13. Retinoic acid according to any one of the preceding claims, wherein said method for treating the skin used to rejuvenate the skin and to remove broken blood vessels, angiomata, scars, lines, wrinkles, pigmentation, and redness in the skin.

14. Retinoic acid according to any one of claims 1 to 12, wherein said method for treating the skin is used to treat acne, warts, cold sores, excema, psoriasis, and infections of the skin.

15. Retinoic acid according to claim 1, wherein said method comprises the steps of:
a) pretreating the skin with topical retinoic acid;
b) producing a chronic wound in the high dermis of the skin by exploding a contaminant on the surface of the skin with laser light, leaving the epidermis intact;
c) treating the skin chronically with said retinoic acid after producing said chronic wound; and
d) maintaining the high dermis in a chronically wounded condition by exploding a contaminant on the surface of the skin with laser light at least once every twelve months, leaving the epidermis intact.

16. Use of retinoic acid in the manufacture of a medicament for use in a method for treating the skin, said method comprising the steps of:
a) pretreating the skin with the retinoic acid;
b) producing a wound in the high dermis of the skin;
c) treating the skin chronically with said retinoic acid after producing said wound in the high dermis; and
d) maintaining the high dermis in a chronically wounded condition;
e) wherein the step of producing a wound in the high dermis is performed by exploding a contaminant on the surface of the skin with laser light.

17. Retinoic acid according to claim 1 or use of retinoic acid according to claim 16, wherein said retinoic acid is applied to the skin 4 to 16 times per month and/or applied topically at a concentration of from 0.05% to 1%.

## Patentansprüche

1. Retinsäure zur Verwendung in einem Verfahren zur Behandlung der Haut, das verfahren umfassend die Schritte:
a) Vorbehandeln der Haut mit Retinsäure;
b) Zufügen einer Wunde in die obere Dermis der Haut;
c) chronisches Behandeln der Haut mit der Retinsäure nach dem Zufügen der Wunde in die obere Dermis; und
d) Aufrechterhalten der oberen Dermis in einem chronisch verwundeten Zustand;
wobei der Schritt des Zufügens einer Wunde in die obere Dermis durch das Explodieren einer Verunreinigung auf der Oberfläche der Haut mit Laserlicht durchgeführt wird.

2. Retinsäure gemäß Anspruch 1, wobei das Verfahren weiter die Schritte des Vorbehandelns und chronischen Behandelns der Haut mit einem Blutgefäß-fördernden Mittel umfasst.

3. Retinsäure gemäß Anspruch 1 oder 2, wobei das Blutgefäßfördernde Mittel Retinsäure ist.

4. Retinsäure gemäß Anspruch 1, wobei die Retinsäure 1- bis 4-mal pro Woche auf die Haut aufgetragen wird.

5. Retinsäure gemäß Anspruch 4, wobei die Retinsäure zweimal pro Woche auf die Haut aufgetragen wird.

6. Retinsäure gemäß den Ansprüchen 4 oder 5, wobei die Retinsäure topisch in einer Konzentration von ungefähr 0,1% angewandt wird.

7. Retinsäure gemäß einem der Ansprüche 3 bis 6, wobei der Vorbehandlungsschritt für zwei Wochen durchgeführt wird.

8. Retinsäure gemäß einem der vorhergehenden Ansprüche, wobei das Zufügen einer chronischen Wunde in die obere Dermis die Epidermis intakt belässt.

9. Retinsäure gemäß einem der vorhergehenden Ansprüche, wobei der Schritt des Zufügens einer chronischen Wunde in die obere Dermis durch Explodieren einer Verunreinigung auf der Haut mit Laserlicht wiederholt innerhalb eines Zeitraums von sechs Monaten des Vorbehandlungsschrittes durchgeführt wird, und/oder der Schritt des Aufrechterhaltens einer chronischen Wunde in der oberen Dermis in einem chronisch verletzten Zustand durch Explodieren einer Verunreinigung auf der Oberfläche der Haut mit Laserlicht mindestens einmal alle zwölf Monate durchgeführt wird.

10. Retinsäure gemäß einem der vorhergehenden Ansprüche, wobei das Zufügen einer Wunde in die obere Dermis in zwei bis zehn Minuten oder in ungefähr vier Minuten beendet ist.

11. Retinsäure gemäß einem der vorhergehenden Ansprüche, wobei die Schritte des Zufügens und Aufrechterhaltens einer chronischen Wunde in der oberen Dermis durch Explodieren einer Verunreinigung auf der Haut mit einem Laserlicht der Wellenlänge von 800 nm bis 1000 nm, einer Pulsfrequenz von 1 bis 20 pro Sekunde, einer Pulsdauer von 0,001 bis 1 Mikrosekunde und einer Fluenz von 1 bis 3 J/cm² durchgeführt wird.

12. Retinsäure gemäß Anspruch 11, wobei das Laserlicht eine Wellenlänge von ungefähr 1064 nm, eine Pulsfrequenz von ungefähr 10 pro Sekunde, eine Pulsdauer von ungefähr 0,01 Mikrosekunde und eine Fluenz von ungefähr 2,5 J/cm² hat, und das Laserlicht optional von einem Q-switched Nd:YAG-Laser produziert wird.

13. Retinsäure gemäß einem der vorhergehenden Ansprüche, wobei das Verfahren zur Behandlung der Haut verwendet wird, um die Haut zu verjüngen und um verletzte Blutgefäße, Angiome, Narben, Linien, Falten, Pigmentierung und Rötung in der Haut zu entfernen.

14. Retinsäure gemäß einem der Ansprüche 1 bis 12, wobei das Verfahren zur Behandlung der Haut zur Behandlung von Akne, Warzen, Fieberblasen, Ekzemen, Psoriasis und Infektionen der Haut verwendet wird.

15. Retinsäure gemäß Anspruch 1, wobei das Verfahren die folgenden Schritte umfasst:
a) topisches Vorbehandeln der Haut mit Retinsäure;
b) Zufügen einer chronischen Wunde in die obere Dermis der Haut durch Explodieren einer Verunreinigung auf der Oberfläche der Haut mit Laserlicht, wobei die Epidermis intakt belassen wird;
c) chronisches Behandeln der Haut mit der Retinsäure nachdem die chronische Wunde zugefügt wurde; und
d) Aufrechterhalten der oberen Dermis in einen chronisch verletzten Zustand durch Explodieren einer Verunreinigung auf der Oberfläche der Haut mit Laserlicht mindestens einmal alle zwölf Monate, wobei die Epidermis intakt belassen wird.

16. Verwendung von Retinsäure zur Herstellung eines Medikaments zur Verwendung in einem Verfahren zur Behandlung der Haut, wobei das Verfahren die folgenden Schritte umfasst:
a) Vorbehandeln der Haut mit Retinsäure;
b) Zufügen einer Wunde in die obere Dermis der Haut;
c) chronisches Behandeln der Haut mit der Retinsäure, nachdem die Wunde in die obere Dermis zugefügt wurde; und
d) Aufrechterhalten der oberen Dermis in einem chronisch verletzten Zustand;
e) wobei der Schritt des Zufügens einer Wunde in die obere Dermis durch Explodieren einer Verunreinigung auf der Oberfläche der Haut mit Laserlicht durchgeführt wird.

17. Retinsäure gemäß Anspruch 1 oder Verwendung von Retinsäure gemäß Anspruch 16, wobei die Retinsäure 4- bis 16-mal pro Monat auf die Haut aufgetragen wird, und/oder topisch in einer Konzentration von 0,05% bis 1% angewendet wird.

## Revendications

1. Acide rétinoïque pour une utilisation dans une méthode de traitement de la peau, ladite méthode comprenant les étapes de :
a) prétraitement de la peau avec de l'acide rétinoïque ;
b) production d'une lésion dans le derme supérieur de la peau ;
c) traitement de la peau de manière chronique avec ledit acide rétinoïque après la production de ladite lésion dans le derme supérieur ; et
d) maintien du derme supérieur dans un état présentant une lésion chronique ;
dans lequel l'étape de production d'une lésion dans le derme supérieur est réalisée en faisant exploser un contaminant à la surface de la peau avec une lumière laser.

2. Acide rétinoïque selon la revendication 1, dans lequel la méthode comprend en outre les étapes de prétraitement et traitement de manière chronique la peau avec un agent favorisant la formation de vaisseaux sanguins.

3. Acide rétinoïque selon la revendication 1 ou 2, dans lequel ledit agent favorisant la formation de vaisseaux sanguins est l'acide rétinoïque.

4. Acide rétinoïque selon la revendication 1, dans lequel ledit acide rétinoïque est appliqué sur la peau 1 à 4 fois par semaine.

5. Acide rétinoïque selon la revendication 4, dans lequel ledit acide rétinoïque est appliqué sur la peau 2 fois par semaine.

6. Acide rétinoïque selon la revendication 4 ou 5, dans lequel ledit acide rétinoïque est appliqué de manière topique à une concentration d'environ 0,1 %.

7. Acide rétinoïque selon l'une quelconque des revendications 3 à 6, dans lequel l'étape de prétraitement est réalisée pendant deux semaines.

8. Acide rétinoïque selon l'une quelconque des revendications précédentes, dans lequel ladite production d'une lésion chronique dans le derme supérieur laisse l'épiderme intact.

9. Acide rétinoïque selon l'une quelconque des revendications précédentes, dans lequel l'étape de production d'une lésion chronique dans le terme supérieur est réalisée en faisant exploser un contaminant à la peau avec une lumière laser de manière répétée dans un délai de six mois après ladite étape de prétraitement et/ou l'étape de maintien d'une lésion chronique dans le derme supérieur dans un état présentant une lésion chronique sur la surface de la peau avec une lumière laser au moins une fois tous les douze mois.

10. Acide rétinoïque selon l'une quelconque des revendications précédentes, dans lequel la production d'une lésion dans le derme supérieur est achevée en deux à dix minutes ou en environ quatre minutes.

11. Acide rétinoïque selon l'une quelconque des revendications précédentes, dans lequel les étapes de production et de maintien d'une lésion chronique dans le derme supérieur sont réalisées en faisant exploser un contaminant à la peau avec une lumière laser ayant une longueur d'onde de 800 nm à 1000 nm, une fréquence d'impulsion de 1 à 20 par seconde, une durée d'impulsion de 0,001 à 1 µs, et une influence de 1 à 3 J/cm².

12. Acide rétinoïque selon la revendication 11, dans lequel ladite lumière laser a une longueur d'onde d'environ 1064 nm, une fréquence d'impulsion d'environ 10 par seconde, une durée d'impulsion d'environ 0,01 µs, une influence d'environ 2,5 J/cm², et facultativement ladite lumière laser est produite par un laser Nd:YAG à impulsions géantes.

13. Acide rétinoïque selon l'une quelconque des revendications précédentes, dans lequel ladite méthode de traitement de la peau utilisée pour rajeunir la peau et pour éliminer les vaisseaux sanguins abîmés, l'angiome, les cicatrices, les lignes, les rides, la pigmentation et la rougeur de la peau.

14. Acide rétinoïque selon l'une quelconque des revendications 1 à 12, dans lequel ladite méthode de traitement de la peau est utilisée pour traiter l'acné, les verrues, les boutons de fièvre, l'eczéma, le psoriasis et les infections de la peau.

15. Acide rétinoïque selon la revendication 1, dans lequel ladite méthode comprend les étapes de :
a) le prétraitement de la peau avec de l'acide rétinoïque topique ;
b) la production d'une lésion chronique dans le derme supérieur de la peau en faisant exploser un contaminant à la surface de la peau avec une lumière laser, laissant l'épiderme intact ;
c) le traitement de la peau de manière chronique avec ledit acide rétinoïque après la production de ladite lésion chronique ; et
d) le maintien du derme supérieur dans un état présentant une lésion chronique en faisant exploser un contaminant à la surface de la peau avec une lumière laser, au moins une fois tous les douze mois, laissant l'épiderme intact.

16. Utilisation d'acide rétinoïque dans la fabrication d'un médicament destiné à une utilisation dans une méthode pour le traitement de la peau, ladite méthode comprenant les étapes de :
a) prétraitement de la peau avec de l'acide rétinoïque ;
b) production d'une lésion dans le derme supérieur de la peau ;
c) traitement de la peau de manière chronique avec ledit acide rétinoïque après la production de ladite lésion dans le derme supérieur ; et
d) maintien du derme supérieur dans un état présentant une lésion chronique ;
e) dans laquelle de l'étape de production d'une liaison dans le derme supérieur est réalisée en faisant exploser un contaminant à la surface de la peau avec une lumière laser.

17. Acide rétinoïque selon la revendication 1 ou l'utilisation d'acide rétinoïque selon la revendication 16, dans laquelle ledit acide rétinoïque est appliqué sur la peau de 4 à 16 fois par mois et/ou est appliquée de manière topique à une concentration de 0,05 % à 1 %.
